# EUROPEAN PATENT APPLICATION

(11) **EP 4 265 291 A1**
(43) Date of publication of application: **25.10.2023**
(21) Application number: 20966238.6
(22) Date of filing: 21.12.2020
(51) Int. Cl.: A61M 16/04

(54) **BREATHING ASSEMBLY SUPPORTING VOICE FUNCTION AND VENTILATION METHOD THEREOF, AND BREATHING MACHINE**

(71) Applicant: Medcaptain Medical Technology Co., Ltd., Shenzhen, Guangdong 518000 (CN)
(72) Inventor: LIU, Jie, Shenzhen, Guangdong 518000 (CN); WEI, Yuchen, Shenzhen, Guangdong 518000 (CN); ZHONG, Yaoqi, Shenzhen, Guangdong 518000 (CN)
(74) Representative: Grünecker Patent- und Rechtsanwälte PartG mbB
(86) International application number: PCT/CN2020/138052
(87) International publication number: WO 2022/133657

(57) **Abstract**

A respiratory assembly supporting a voice function, and a ventilation method and a ventilator thereof. The respiratory assembly includes a detecting module configured to detect a mouth pressure and an expiratory airway pressure of a patient, an inspiratory module configured to provide gas to the patient during inspiration, and an expiratory module configured to expel the gas generated by the patient's expiration, and control an expiratory branch pressure to be greater than the mouth pressure according to the mouth pressure detected by the detecting module, which causes the expiratory airway pressure to rise due to lung contraction during the patient's expiration, causing gas in the expiratory airway of the patient to flow toward a mouth, and the flowing gas to drive patient's vocal cords to vibrate and vocalize. Compared to the speaking valve in the prior art, the respiratory assembly in the present disclosure does not need to be dismantled, which avoids wear and tear of components, thus reducing costs. Besides, since it does not need to be dismantled, it is easy to operate clinically and is conducive to quickly meeting the patient's vocal requirements.

## Description

### TECHNICAL FIELD

The present disclosure generally relates to the field of medical technologies, and more particularly, to a respiratory assembly supporting a voice function, a ventilation method and ventilator thereof.

### BACKGROUND

Tracheotomy is a procedure in which the patient's cervical trachea is cut and a cannula is placed to resolve respiratory distress and respiratory dysfunction. Patients who undergo tracheotomy clinically need to be intubated for assisted breathing and gas exchange with the outside world. Gas does not flow through the upper airway and vocal cords, and no sound can be made, which hinders doctor-patient communication. Speaking valves are now used clinically to help patients vocalize, and the principle is that a one-way valve is used to prohibit the passage of exhaled gas through the expiratory branch of the tracheal intubation and pass through the upper airway, allowing the vocal cords to vibrate and vocalize.

However, when using the speaking valve, the cannula needs to be removed to mount the speaking valve, and the speaking valve needs to be removed after use. Some patients can adapt to the speaking valve quickly, while others need to train to gradually increase the duration of wearing the speaking valve. Repeated disassembly of the speaking valve can result in complex clinical operations and wear and tear of the components, leading to increased costs. There are also some speaking valves designed to be embedded inside the cannula, but they still need to be remounted, and the operation steps are not simplified. Besides, they need to conform to the respiratory mechanic's design, which increases the product production cost. Therefore, the conventional speaking valves suffer from the problem of inconvenient use.

Therefore, it becomes critical to design a respiratory assembly that does not require repeated disassembly to replace the speaking valve.

### SUMMARY

An objective of the present disclosure is to provide a respiratory assembly supporting a voice function, a ventilation method, and a ventilator thereof. The respiratory assembly is capable of replacing the speaking valve, and without disassembly, which avoids wear and tear of components, reduces costs, and is clinically simple to operate.

In order to achieve the objective of the present disclosure, the present disclosure provides the following technical solutions.

According to a first aspect, the present disclosure provide a respiratory assembly supporting a vocal function, comprising a detecting module, the detecting module being configured to detect a mouth pressure and an expiratory airway pressure of a patient; an inspiratory module, the inspiratory module being configured to provide gas to the patient during inspiration; an expiratory module, the expiratory module comprising an expiratory branch connected to an expiratory airway of the patient; the expiratory module being configured to expel gas generated by patient's expiration; the expiratory module being further configured to control an expiratory branch pressure to be greater than the mouth pressure according to the mouth pressure detected by the detecting module, which causes the expiratory airway pressure to rise due to lung contraction during the patient's expiration, causing gas in the expiratory airway of the patient to flow toward a mouth, and the flowing gas to drive patient's vocal cords to vibrate and vocalize.

In an embodiment, the expiratory module comprises a regulating member provided on the expiratory branch; the regulating member is configured to regulate a difference between the expiratory branch pressure and the mouth pressure to a first predetermined value, wherein the first predetermined value is greater than zero.

In an embodiment, the respiratory assembly further comprises an alarm module, and the alarm module is configured to perform an alarm action when the mouth pressure or the expiratory airway pressure is greater than a second predetermined value.

In an embodiment, the expiratory module is further configured to control the expiratory branch pressure to be the same as the mouth pressure to stagnate the gas in the expiratory branch.

In an embodiment, the expiratory module is further configured to control the expiratory branch, wherein the cannula is configured to insert into the expiratory airway; the balloon is provided at a periphery of the cannula, and the detecting module is further configured to obtain a pressure inside the balloon in real time.

In an embodiment, the alarm module is further configured to perform an alarm action when the pressure inside the balloon is greater than a third predetermined value.

In an embodiment, the respiratory assembly further comprises an analysis module; wherein the analysis module is configured to analyze the expiratory airway pressure and the mouth pressure obtained by the detecting module to determine whether the patient is exhaling or inhaling, and to record the number of respiratory cycles of the patient.

According to a second aspect, the present disclosure further provides a ventilator, comprising a respiratory assembly of any one of embodiments in the first aspect.

According to a third aspect, the present disclosure further provides a ventilation method of a respiratory assembly supporting a voice function, wherein the respiratory assembly comprises an expiratory branch connected to an expiratory airway of a patient, and the ventilation method of the respiratory assembly comprises: detecting a mouth pressure and an expiratory airway pressure of the patient; providing gas to the patient during inspiration when the patient inhales; and expelling gas generated by patient's expiration when the patient exhales, or controlling the expiratory branch pressure to be greater than the mouth pressure, which causes the expiratory airway pressure to rise due to lung contraction during the patient's expiration, causing gas in the expiratory airway of the patient to flow toward a mouth, and the flowing gas to drive patient's vocal cords to vibrate and vocalize.

In an embodiment, the respiratory assembly further comprises a regulating member provided on the expiratory branch; the step of expelling gas generated by patient's expiration when the patient exhales or controlling the expiratory branch pressure to be greater than the mouth pressure, which causes the expiratory airway pressure to rise due to lung contraction during the patient's expiration, causing gas in the expiratory airway of the patient to flow toward the mouth, and the flowing gas to drive patient's vocal cords to vibrate and vocalize, comprises:
regulating, by the regulating member, a difference between the expiratory branch pressure and the mouth pressure to a first predetermined value, wherein the first predetermined value is greater than zero.

In an embodiment, before regulating, by the regulating member, the difference between the expiratory branch pressure and the mouth pressure to the first predetermined value, the method further comprises:
performing an alarm action when the mouth pressure or the expiratory airway pressure is greater than a second predetermined value.

In an embodiment, before performing the alarm action when the mouth pressure or the expiratory airway pressure is greater than the second predetermined value, the method further comprises:
filling the expiratory branch with gas by a plurality of expirations when the patient is at an initial expiration.

In an embodiment, before filling the expiratory branch with gas by a plurality of expirations when the patient is at an initial expiration, the method further comprises:
regulating, by the regulating member, the expiratory branch pressure so that the expiratory branch pressure is the same as the mouth pressure.

In an embodiment, the method further comprises:
regulating, by the regulating member, the expiratory branch pressure to zero, after the patient has breathed a predetermined number of cycles in a voice ventilation mode.

In an embodiment, the respiratory assembly further comprises a tracheal cannula and a balloon, wherein the tracheal cannula is threaded through the expiratory airway of the patient and the balloon is provided at a periphery of the tracheal cannula; the balloon is filled with gas and blocks the expiratory airway when a voice ventilation mode is not turned on; before detecting the mouth pressure and the expiratory airway pressure of the patient, the method further comprises:
expelling gas in the balloon and turning on the voice ventilation mode to obtain a pressure inside the balloon in real time.

In an embodiment, after expelling the gas in the balloon and turning on the voice ventilation mode to obtain the pressure inside the balloon in real time, the method further comprises:
performing an alarm action when the pressure inside the balloon is greater than a third predetermined value.

By detecting the mouth pressure and the expiratory airway pressure of the patient by the detecting module, the expiratory module can make the expiratory branch pressure of the patient greater than the mouth pressure when the patient needs to vocalize, so as to provide the patient with a respiratory mode capable of vocalizing. When the patient does not need to vocalize, the inspiratory module and the expiratory module can provide the patient with a respiratory mode in which breathing is not dependent on the outside world. Compared to the speaking valve in the prior art, the respiratory assembly in the present disclosure does not need to be dismantled, which can avoid wear and tear of components, thus reducing the cost. Besides, since the respiratory assembly does not need to be dismantled, the clinical operation is simple, which is conducive to quickly meeting the patient's vocalization requirements.

### BRIEF DESCRIPTION OF THE DRAWINGS

In order to illustrate the technical solutions in the embodiments of the present disclosure or in the prior art more clearly, the accompanying drawings to be used in the description of the embodiments or the prior art will be briefly described below. Obviously, the accompanying drawings in the following description are some embodiments of the present disclosure, for a person having ordinary skill in the art, other accompanying drawings can be obtained without creative work.
FIG 1 is a schematic diagram of a respiratory assembly in operation according to an embodiment of the present disclosure.
FIG 2 is a schematic diagram of a respiratory airway of the patient while using a cannula and a balloon.
FIG 3 is a schematic diagram of a gas flow during an inspiration of the patient with the respiratory assembly in an assisted ventilation mode.
FIG 4 is a schematic diagram of a gas flow during an expiration of the patient with the respiratory assembly in the assisted ventilation mode.
FIG 5a is a schematic diagram of a gas flow during the inspiration of the patient with the respiratory assembly in a voice ventilation mode according to an embodiment of the present disclosure.
FIG 5b is a schematic diagram of a gas flow during the inspiration of the patient with the respiratory assembly in the voice ventilation mode according to another embodiment of the present disclosure.
FIG 5c is a schematic diagram of a gas flow during the inspiration of the patient with the respiratory assembly in the voice ventilation mode according to yet another embodiment of the present disclosure.
FIG 6 is a flowchart of a ventilation method of the respiratory assembly in the voice ventilation mode according to an embodiment of the present disclosure.
FIG 7 is a flowchart of an alarm of the pressure in the balloon of the respiratory assembly according to an embodiment of the present disclosure.

### DETAILED DESCRIPTION

The technical solutions in the embodiments of the present disclosure will be described clearly and completely as follows in conjunction with the accompanying drawings in the embodiments of the present disclosure. Obviously, the embodiments described are a part of rather than all of the embodiments of the present disclosure. Based on the embodiments in the present disclosure, all other embodiments obtained by a person having ordinary skill in the art without creative labor fall within the protection scope of the present disclosure.

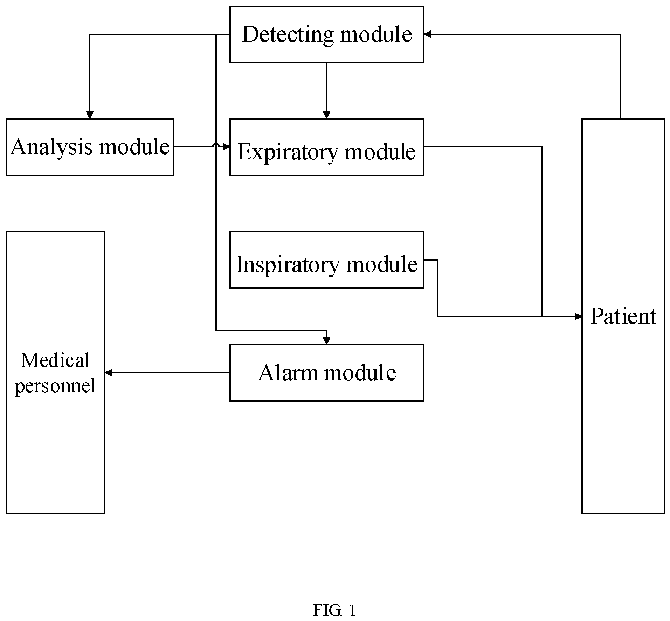

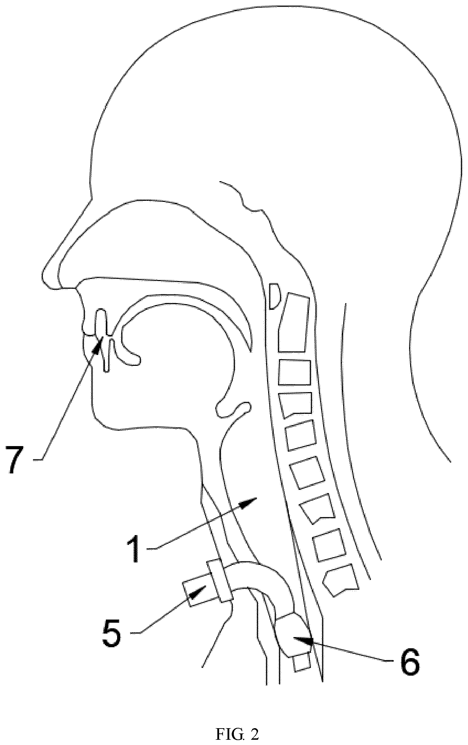

As shown in FIGS. 1 to 3, embodiments of the present disclosure provide a respiratory assembly supporting a voice function. The respiratory assembly may be applied to a ventilator, oxygen machine, and other assisted breathing devices, in which the respiratory assembly may be used as a component or an external portion. The respiratory assembly is preferably applied to an invasive ventilator. The respiratory assembly includes a detecting module, an inspiratory module, and an expiratory module.

The detecting module is configured to detect a mouth pressure (Pmouth) and an expiratory airway pressure of a patient.

The inspiratory module is configured to provide gas to the patient during inspiration.

The expiratory module includes an expiratory branch (2) connected to an expiratory airway (1). The expiratory module is configured to expel the gas generated by the patient's expiration, and the expiratory module is further configured to control the expiratory branch pressure (Pexp) to be greater than the mouth pressure according to the mouth pressure detected by the detecting module, which causes the expiratory airway pressure to rise due to lung contraction during the patient's expiration, causing gas in the expiratory airway of the patient to flow toward a mouth, and the flowing gas to drive patient's vocal cords to vibrate and vocalize.

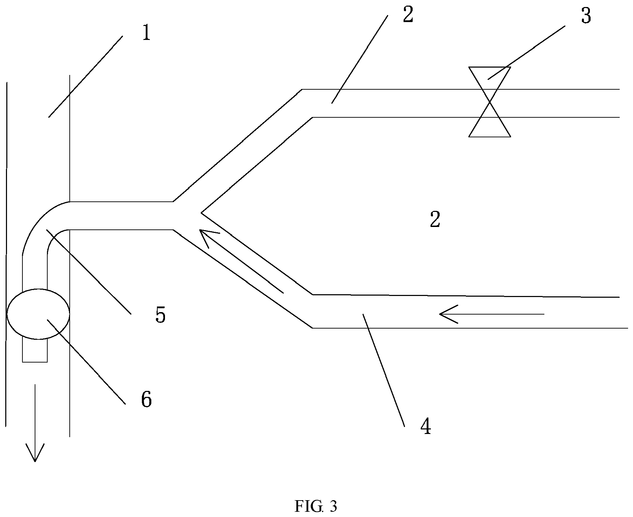

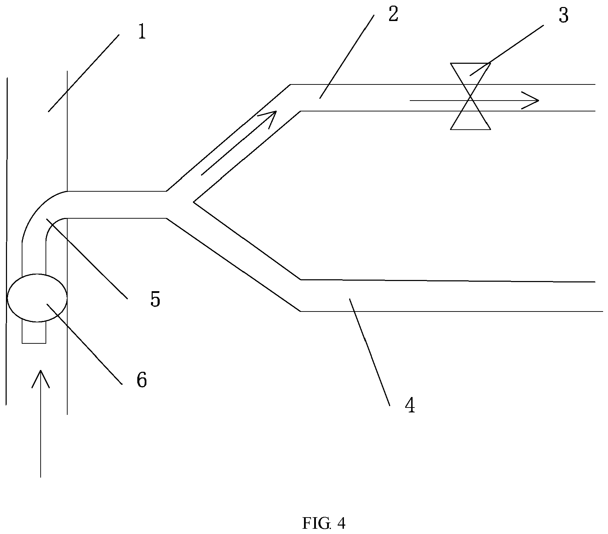

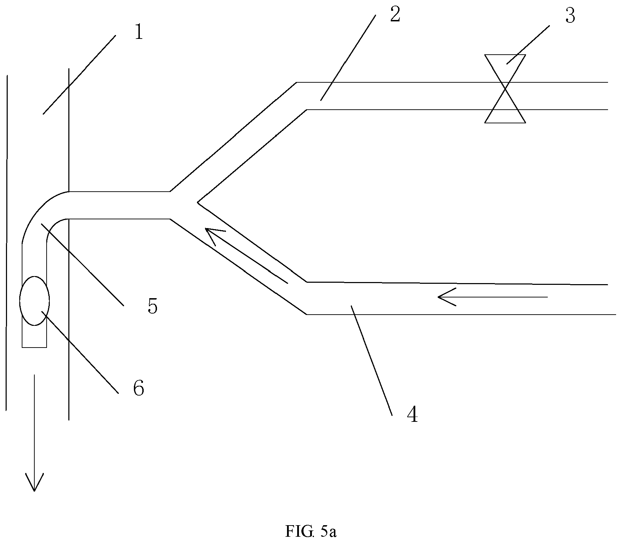

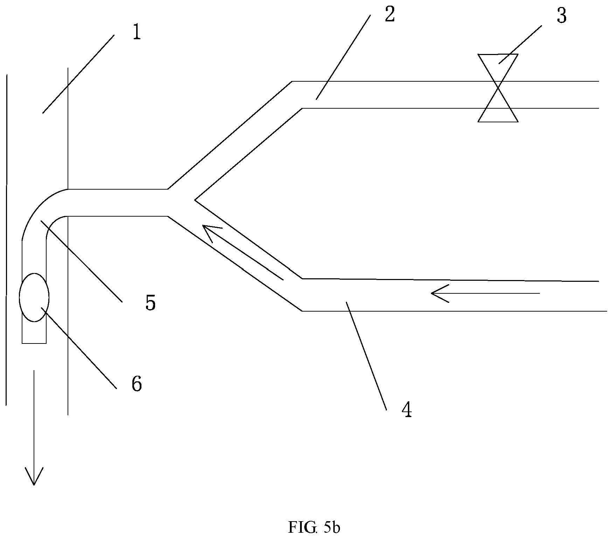

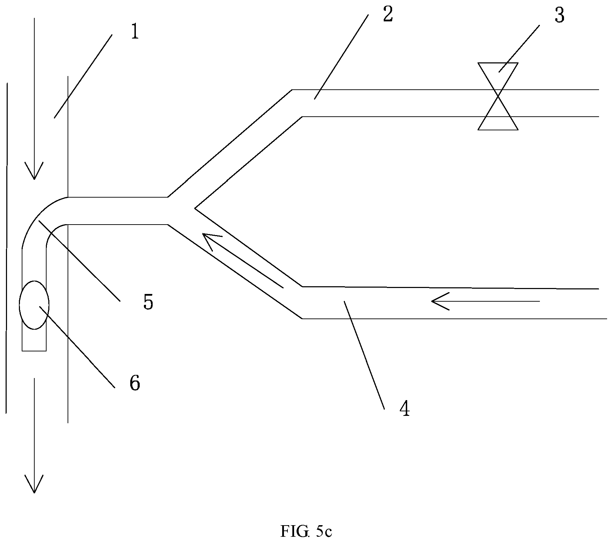

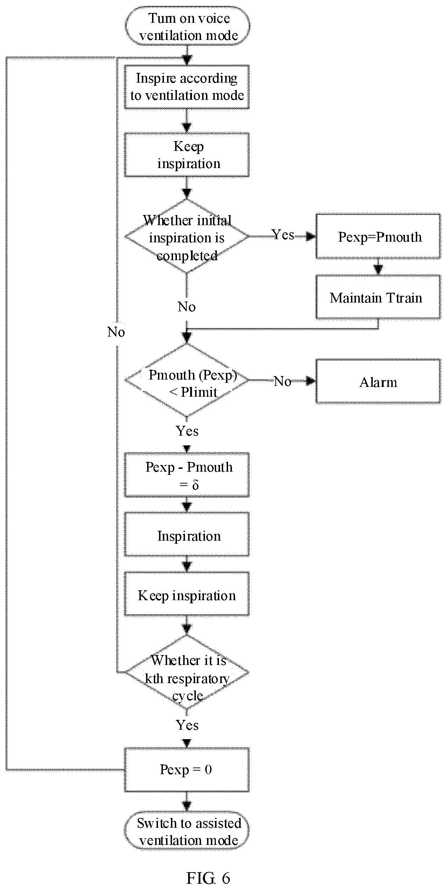

Specifically, the mouth pressure is the pressure in the mouth (oral cavity) during the patient's expiration, the expiratory airway pressure is the pressure in the expiratory airway (lower respiratory airway) during the patient's expiration, and the expiratory branch pressure is a pressure provided by a Positive End Expiratory Pressure value (PEEP valve). Preferably, the detecting of the detecting module is real-time, allowing for real-time acquisition of mouth pressure and expiratory airway pressure. It can be understood that, as shown in FIGS. 3 and 4, when the patient does not need to vocalize, gas can be drawn in through the inspiratory module and exhaled from the expiratory module, with both inspiration and expiration done through the respiratory assembly, which contributes to patient comfort. Regarding inspiration, as shown in FIG 5a, the inspiratory module can be used as the only source of gas for the patient when the patient needs to vocalize, and the patient can only inhale the gas provided by the inspiratory module. As shown in FIG 5b, the inspiratory module can also be turned off, allowing the patient to inhale gas only from the outside through the mouth. As shown in FIG 5c, the inspiratory module may also act as an assisted gas delivery, replenishing the gas when the patient is not inhaling enough gas from the outside world, allowing the patient to fully complete the pulmonary cycle. Regarding expiration, as shown in FIG 6, under the control of the expiratory module, the pressure in the expiratory branch is greater than the mouth pressure. When the patient performs spontaneous expiration, a small portion of the gas is expelled from the expiratory module due to the expiratory airway pressure (the expiratory module needs to release some of the gas to control the expiratory airway pressure), and the majority of the gas is expelled from the expiratory airway through the mouth, so that most of the gas can reach the mouth along the expiratory airway. The gas passes through the vocal cords in the middle of the laryngeal cavity, causing the vocal cords to vibrate and allowing the patient to vocalize, thus enabling the voice function. The expiratory module is configured to control the expiratory airway pressure to be greater than the mouth pressure, to enable the patient to vocalize in the voice ventilation mode, and the expiratory module is configured to expel the patient's exhaled gas in the assisted ventilation mode.

The mouth pressure and the expiratory airway pressure of the patient are detected by the detecting module. When the patient needs to vocalize, the expiratory module can make the expiratory airway pressure greater than the mouth pressure to provide the patient with a respiratory mode capable of vocalizing. When the patient does not need to vocalize, the inspiratory module and the expiratory module can provide the patient with a respiratory mode that is not dependent on the outside world. Compared to the speaking valve in the related art, the respiratory assembly in the present disclosure does not need to be dismantled, which can avoid wear and tear of components, thus reducing costs. Besides, since it does not need to be dismantled, the clinical operation is simple, which is conducive to quickly meeting the patient's vocalization requirements.

In one embodiment, as shown in FIGS. 3 and 6, the expiratory module further includes a regulating member (3) provided on the expiratory branch, which is configured to regulate a difference between the expiratory branch pressure and the mouth pressure to a first predetermined value (δ). Specifically, the first predetermined value is greater than zero, optionally 0.1 cm H2O to 1 cmH2O. The first predetermined value is a parameter according to the patient's physical qualities such as age, body mass index (BMI), and physical condition. When the difference between the expiratory branch pressure and the mouth pressure is too large, it tends to lead to excessive pressure in the expiratory airway, which can lead to lung injury in patients. When the difference between expiratory branch pressure and mouth pressure is too small, it tends to cause most of the gas to be exhaled from the expiratory branch without passing through the expiratory airway, which is not conducive to the patient's vocalization. By providing the regulating member inside the expiratory branch, the difference between the expiratory airway pressure and the mouth pressure is regulated to the first predetermined value by the regulating member, so that the patient has a more suitable pressure difference between the expiratory branch and the mouth during expiration, i.e., it will not hurt the patient's lungs and will not affect the patient's vocalization.

In addition, the positive end expiratory pressure (PEEP) needs to be set to zero by the regulating member before the voice ventilation mode is turned on. The inspiratory module includes an inspiratory branch, which is mainly configured to deliver gas to the patient. An inspiratory valve (not shown) may be provided on the inspiratory branch and an expiratory valve (not shown) may be provided on the expiratory branch.

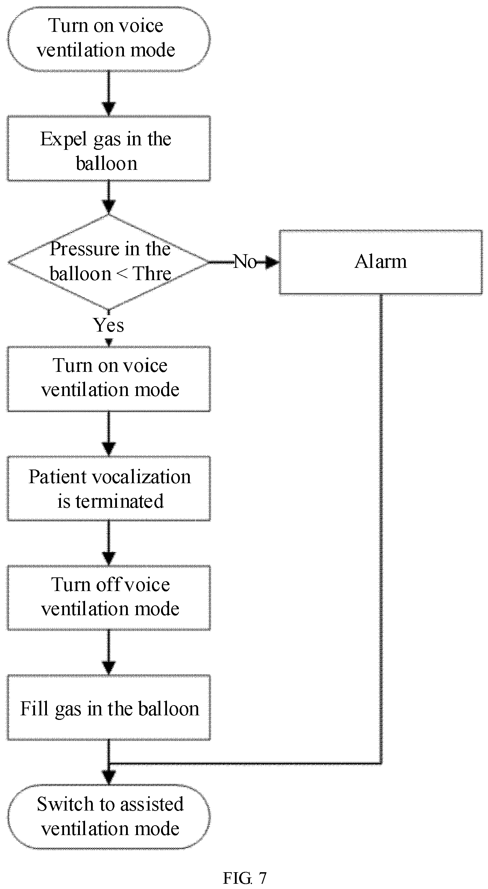

In one embodiment, as shown in FIGS. 1 and 7, the respiratory assembly further includes an alarm module configured to perform an alarm action when the mouth pressure or the expiratory airway pressure is greater than a second predetermined value (Plimit). Specifically, the second predetermined value is a safe limiting pressure according to the patient's physical qualities such as age, BMI, and physical condition. It can be understood that patients are prone to respiratory distress or lung injury if the mouth pressure or the expiratory airway pressure is too high. Generally, the second predetermined value is generally no more than 60 cmH2O. By setting the alarm module, an alarm action is performed by the alarm module when the mouth pressure or the expiratory airway pressure is greater than the second predetermined value, so that medical personnel can detect abnormal conditions of patients in time and take relevant measures as early as possible, which is conducive to improving the safety of patient's respiration.

In one embodiment, as shown in FIG 1, the respiratory assembly further includes an analysis module, which is configured to analyze the expiratory airway pressure and the mouth pressure obtained by the detecting module to determine whether the patient is exhaling or inhaling and to record the number of respiratory cycles (k) of the patient. It can be understood that after the voice ventilation mode is turned on (the expiratory module controls the expiratory branch pressure to be greater than the mouth pressure), the patient is prone to various symptoms of discomfort as the number of respiratory cycles of the patient increases. The number of respiratory cycles that each patient can tolerate is different and may be set according to the patient's physical qualities such as age, BMI, and physical condition.

In one embodiment, as shown in FIGS. 1 and 6, the expiratory module is further configured to control the respiratory branch pressure to zero. The number of respiratory cycles of the patient is obtained through the analysis module so that when the patient reaches the limit of the number of respiratory cycles, the patient's discomfort is eliminated by regulating the expiratory branch pressure to zero, allowing the gas in the expiratory airway to be released entirely through the expiratory module.

In one embodiment, as shown in FIGS. 1 and 6, after releasing gas from the expiratory airway through the expiratory module, the expiratory branch needs to be refilled with gas. It can be understood that after the patient has reached the limited number of respiratory cycles and has set the expiratory branch pressure to zero, there is virtually no gas in the expiratory branch. When the patient needs to re-vocalize, the expiratory branch pressure is regulated by setting the PEEP valve so that the exhaled gas fills the expiratory branch first (this process is referred to as "Ttrain" and is usually completed within 3 breaths). Once the filling is complete, most of the gas generated by the patient's expiration is expelled from the mouth, allowing the vocal cords to vibrate sufficiently to vocalize.

In other embodiments, the expiration branch may also be filled with gas via the inspiratory module to reduce the Ttrain time.

In one embodiment, as shown in FIGS. 1 and 6, the expiratory module is further configured to control the expiration branch pressure to be equal to the mouth pressure, allowing for gas stagnation in the expiration branch. It can be understood that if the expiratory airway pressure is greater than the mouth pressure, the gas filled to the expiratory airway tends to escape from the mouth, which is not conducive to the rapid completion of gas filling of the expiratory airway. If the expiratory branch pressure is less than the mouth pressure, the gas in the expiratory branch tends to flow into the lungs, which is also not conducive to filling the expiratory branch with gas.

In one embodiment, as shown in FIGS. 3 and 5a, the respiratory assembly further includes a cannula (5) and a balloon (6). The cannula is inserted into the expiratory airway and the balloon is provided at the periphery of the cannula. The detecting module is further configured to obtain the pressure inside the balloon in real time. Specifically, both the expiratory module and the inspiratory module are connected to the cannula. The patient's exhaled gas may enter the expiratory module from the cannula, and the patient's inhaled gas may enter the expiratory airway from the inspiratory module through the cannula. In general, before entering the voice ventilation mode, the gas in the balloon needs to be released to allow the pressure inside the balloon to decrease (the balloon is no longer blocking the expiratory airway). The pressure inside the balloon is detected by the detecting module to facilitate the timely detection of abnormal balloon pressure (usually caused by incomplete balloon deflation) and the early management of the situation (shutting down the voice ventilation mode, etc.) to reduce the risk of patient asphyxia.

Specifically, the cannula is 4 mm to 12 mm in size inside the body and 15 mm outside the body, and it is also easier to fill the cannula when filling the gas in the expiratory branch.

At present, the conventional detachable speaking valve may be provided with a vent hole, which will automatically open to leak part of the gas when the internal pressure of the device is too large. However, since the conventional speaking valve devices are all detachable components, it is not possible to arrange an alarm, which makes it difficult to realize a real-time alarm when the gas inside the balloon is not completely eliminated, further reducing the risk of patient asphyxia.

In one embodiment, as shown in FIGS. 1 and 7, the alarm module is further configured to perform an alarm action when the pressure inside the balloon is greater than a third predetermined value. It can be understood that, during the voice ventilation mode, if an unexpected situation occurs that makes the pressure inside the balloon increase, it will be detrimental to the patient's breathing and the patient has a safety risk. By setting the alarm module, when the pressure inside the balloon exceeds the third predetermined value, the alarm module makes an alarm, which is beneficial for the medical personnel to rescue the patient the first time. Since there is no need to set the speaking valve, the pressure inside the balloon is easier to obtain, so it is also convenient for the alarm module to make an alarm.

In addition, the alarm action may be a flashing light, a sharp sound, or alarm information sent to the medical personnel's smartphone, tablet, personal digital assistance, intercom, desktop computer, and other electronic devices. It may be set according to the needs of specific scenarios.

Embodiments of the present disclosure further provide a respirator, which includes the respiratory assembly provided in the present disclosure. Specifically, the respirator is preferably invasive. Assisted inspiratory modes of the ventilator include, but are not limited to spontaneous respiratory mode (SPONT), pressure-controlled ventilation mode (PCV+), pressure-controlled and spontaneous ventilation mode (PSIMV+), and continuous positive airway pressure ventilation mode (CPAP). By incorporating the respiratory assembly provided in the present disclosure, the ventilator is capable of performing a voice ventilation mode (capable of vocalization) as well as an assisted ventilation mode (not capable of vocalization). Switching between the two modes without disassembling the components, thereby avoiding wear and tear of components and reducing costs. Besides, since no disassembly is required, the clinical operation is simple and facilitates rapid satisfaction of the patient's vocalization requirements.

As shown in FIG 7, embodiments of the present disclosure further provide a ventilation method of a respiratory assembly supporting a voice function. The respiratory assembly includes an expiratory branch connected to the expiratory airway, and the ventilation method of the respiratory assembly includes the following steps.

A mouth pressure and an expiratory airway pressure of a patient are detected.

The gas is provided to the patient during the patient's inspiration.

The gas generated by the patient's expiration is expelled, or the expiration branch pressure is controlled to be greater than the mouth pressure, which causes the expiratory airway pressure to rise due to lung contraction during the patient's expiration, causing gas in the expiratory airway of the patient to flow toward a mouth, and the flowing gas to drive patient's vocal cords to vibrate and vocalize.

Specifically, the detection of the mouth pressure and the expiratory airway pressure of the patient is preferably real-time, so that the mouth pressure and the expiratory airway pressure can be obtained in real time. By detecting the mouth pressure and the expiratory airway pressure in real time, when the patient needs to vocalize, the expiratory branch pressure is regulated to be greater than the mouth pressure. At the same time, when the patient performs spontaneous expiration, the expiratory airway pressure rises due to lung contraction, and gas passes through the expiratory airway, to provide the patient with a respiratory mode capable of vocalizing. When the patient does not need to vocalize, a non-dependent external respiratory mode is provided to the patient. Compared to the speaking valves in the related art, the switching of the ventilation method of the present disclosure does not require removal, which avoids wear and tear of components, thereby reducing costs. Besides, since it does not require removal, it is clinically simple and facilitates rapid satisfaction of the patient's vocal requirements.

In one embodiment, as shown in FIG 7, the respiratory assembly includes a regulating member provided on the expiratory branch. The step of expelling gas generated by the patient's expiration or controlling the expiratory branch pressure to be greater than the mouth pressure, which causes gas in the expiratory airway of the patient to flow toward the mouth, and the flowing gas to drive the patient's vocal cords to vibrate and vocalize includes the following steps.

A difference between the expiratory branch pressure and the mouth pressure is regulated to a first predetermined value by the regulating member. Specifically, the first predetermined value is greater than zero.

By regulating the difference between the expiratory branch pressure and the mouth pressure to the first predetermined value, the patient has a more suitable pressure difference between the expiratory airway and the mouth during expiration, i.e., the patient's lungs will not be injured and the patient's vocalization will not be affected.

In one embodiment, as shown in FIG 7, before regulating, by the regulating member, the difference between the expiratory branch pressure and the mouth pressure to the first predetermined value, the method further includes the following step.

An alarm action is performed when the mouth pressure or the expiratory airway pressure is greater than a second predetermined value.

Specifically, when both the mouth pressure and the expiratory airway pressure are less than or equal to the second predetermined value, the next step is performed (regulating, by the regulating member, the difference between the expiratory branch pressure and the mouth pressure to the first predetermined value).

By performing an alarm action when the mouth pressure or the expiratory airway pressure is greater than the second predetermined value, medical personnel can detect abnormal conditions in time and take relevant measures as soon as possible, which is conducive to improving the safety of patient's breathing.

In one embodiment, as shown in FIG 7, the respiratory branch pressure is made to zero when the patient has reached a preset number of respiratory cycles in the voice ventilation mode. Specifically, the kth respiratory cycle is based on the completion of the kth expiratory hold to the end of expiration. By obtaining the number of respiratory cycles of the patient, it facilitates setting the expiratory branch pressure to zero when the patient reaches the limit of the respiratory cycles to release gas from the expiratory airway, thus making the patient comfortable.

In one embodiment, as shown in FIG 7, before performing an alarm action when the mouth pressure or the expiratory airway pressure is greater than a second predetermined value, the method further includes the following step.

The expiratory branch is filled with gas by multiple expirations when the patient is at an initial expiration.

Specifically, when the patient is a non-first expiration after the initial expiration, the next step is performed (determining the magnitude of the mouth pressure and the second predetermined value).

By filling the expiratory branch with gas through multiple expirations, the gas fills the expiratory branch and most of the gas generated by the patient's expiration is expelled from the mouth, which facilitates adequate vibration of the vocal cords for vocalization.

In one embodiment, as shown in FIG 7, before filling the expiratory branch with gas by multiple expirations when the patient is at an initial expiration, the method further includes the following step.

The expiration branch pressure is regulated by the PEEP valve so that the expiration branch pressure is the same as the mouth pressure.

Specifically, the initial expiration begins when the inspiration is held until the initial inspiration is completed. It can be understood that if the expiratory branch pressure is greater than the mouth pressure, the gas filling to the expiratory branch tends to escape from the mouth, which is not conducive to the rapid completion of gas filling of the expiratory branch. If the expiratory airway pressure is less than the mouth pressure, it is easy to make the gas from the expiratory airway flow into the lungs, which is also not conducive to filling the expiratory airway with gas.

In one embodiment, as shown in FIG 7, the ventilator further includes a tracheal cannula and a balloon, the tracheal cannula is threaded through the patient's expiratory airway, and the balloon is provided at the periphery of the tracheal cannula. When the voice ventilation mode (assisted ventilation mode) is not turned on, the balloon is filled with gas and blocks the expiratory airway. Before detecting the mouth pressure and the expiratory airway pressure, the method further includes the following step.

The gas in the balloon is expelled to turn on the voice ventilation mode, and the pressure inside the balloon is obtained in real time.

The pressure inside the balloon is detected to facilitate the timely detection of abnormalities in the pressure inside the balloon and early relevant treatment (turning off the voice ventilation mode, etc.).

In one embodiment, as shown in FIG 7, after expelling the gas in the balloon to turn on the voice ventilation mode and obtaining the pressure inside the balloon in real time, the method further includes the following step.

An alarm action is performed when the pressure inside the balloon is greater than a third predetermined value (Thre).

By performing the alarm action when the pressure inside the balloon exceeds the third predetermined value, it facilitates the first-time rescue of the patient by the medical personnel.

Disclosed above are only some better embodiments of the present disclosure, which certainly cannot be used to limit the scope of the claims of the present disclosure. It can be understood for a person having ordinary skill in the art that all or part of the processes for implementing the above embodiments, and equivalent changes made in accordance with the claims of the present disclosure, shall fall within the scope covered by the present disclosure.

## Claims

1. A respiratory assembly supporting a vocal function, comprising:
a detecting module, the detecting module being configured to detect a mouth pressure and an expiratory airway pressure of a patient;
an inspiratory module, the inspiratory module being configured to provide gas to the patient during inspiration; and
an expiratory module, the expiratory module comprising an expiratory branch connected to an expiratory airway of the patient; the expiratory module being configured to expel gas generated by patient's expiration; the expiratory module being further configured to control an expiratory branch pressure to be greater than the mouth pressure according to the mouth pressure detected by the detecting module, which causes the expiratory airway pressure to rise due to lung contraction during the patient's expiration, causing gas in the expiratory airway of the patient to flow toward a mouth, and the flowing gas to drive patient's vocal cords to vibrate and vocalize.

2. The respiratory assembly of claim 1, wherein the expiratory module comprises a regulating member provided on the expiratory branch; the regulating member is configured to regulate a difference between the expiratory branch pressure and the mouth pressure to a first predetermined value, wherein the first predetermined value is greater than zero.

3. The respiratory assembly of claim 2, further comprising an alarm module, wherein the alarm module is configured to perform an alarm action when the mouth pressure or the expiratory airway pressure is greater than a second predetermined value.

4. The respiratory assembly of claim 1, wherein the expiratory module is further configured to control the expiratory branch pressure to be the same as the mouth pressure to stagnate the gas in the expiratory branch.

5. The respiratory assembly of claim 1, wherein the expiratory module is further configured to control the expiratory branch pressure to zero.

6. The respiratory assembly of claim 3, further comprising a cannula and a balloon, wherein the cannula is configured to insert into the expiratory airway; the balloon is provided at a periphery of the cannula, and the detecting module is further configured to obtain a pressure inside the balloon in real time.

7. The respiratory assembly of claim 6, wherein the alarm module is further configured to perform an alarm action when the pressure inside the balloon is greater than a third predetermined value.

8. The respiratory assembly of claim 1, further comprising an analysis module; wherein the analysis module is configured to analyze the expiratory airway pressure and the mouth pressure obtained by the detecting module to determine whether the patient is exhaling or inhaling and to record the number of respiratory cycles of the patient.

9. A ventilator, comprising a respiratory assembly of any one of claims 1 to 8.

10. A ventilation method of a respiratory assembly supporting a voice function, wherein the respiratory assembly comprises an expiratory branch connected to an expiratory airway of a patient, and the ventilation method of the respiratory assembly comprises:
detecting a mouth pressure and an expiratory airway pressure of the patient;
providing gas to the patient during patient's inspiration; and
expelling gas generated by patient's expiration, or controlling the expiratory branch pressure to be greater than the mouth pressure, which causes the expiratory airway pressure to rise due to lung contraction during the patient's expiration, causing gas in the expiratory airway of the patient to flow toward a mouth, and the flowing gas to drive patient's vocal cords to vibrate and vocalize.

11. The ventilation method of the respiratory assembly of claim 10, wherein the respiratory assembly further comprises a regulating member provided on the expiratory branch; the step of expelling gas generated by patient's expiration or controlling the expiratory branch pressure to be greater than the mouth pressure, which causes the expiratory airway pressure to rise due to lung contraction during the patient's expiration, causing gas in the expiratory airway of the patient to flow toward the mouth, and the flowing gas to drive patient's vocal cords to vibrate and vocalize, comprises:
regulating, by the regulating member, a difference between the expiratory branch pressure and the mouth pressure to a first predetermined value, wherein the first predetermined value is greater than zero.

12. The ventilation method of the respiratory assembly of claim 11, wherein before regulating, by the regulating member, the difference between the expiratory branch pressure and the mouth pressure to the first predetermined value, the method further comprises:
performing an alarm action when the mouth pressure or the expiratory airway pressure is greater than a second predetermined value.

13. The ventilation method of the respiratory assembly of claim 12, wherein before performing the alarm action when the mouth pressure or the expiratory airway pressure is greater than the second predetermined value, the method further comprises:
filling the expiratory branch with gas by a plurality of expirations when the patient is at an initial expiration.

14. The ventilation method of the respiratory assembly of claim 13, wherein before filling the expiratory branch with gas by a plurality of expirations when the patient is at an initial expiration, the method further comprises:
regulating, by the regulating member, the expiratory branch pressure so that the expiratory branch pressure is the same as the mouth pressure.

15. The ventilation method of the respiratory assembly of claim 12, further comprising:
regulating, by the regulating member, the expiratory branch pressure to zero, after the patient has breathed a predetermined number of cycles in a voice ventilation mode.

16. The ventilation method of the respiratory assembly of claim 10, wherein the respiratory assembly further comprises a tracheal cannula and a balloon, wherein the tracheal cannula is threaded through the expiratory airway of the patient and the balloon is provided at a periphery of the tracheal cannula; the balloon is filled with gas and blocks the expiratory airway when a voice ventilation mode is not turned on; before detecting the mouth pressure and the expiratory airway pressure of the patient, the method further comprises:
expelling gas in the balloon and turning on the voice ventilation mode to obtain a pressure inside the balloon in real time.

17. The ventilation method of the respiratory assembly of claim 16, wherein after expelling the gas in the balloon and turning on the voice ventilation mode to obtain the pressure inside the balloon in real time, the method further comprises:
performing an alarm action when the pressure inside the balloon is greater than a third predetermined value.
